Europäisches Patentamt

European Patent Office

Office européen des brevets

(19)

(11) Publication number: **0 387 777**
**A2**

# EUROPEAN PATENT APPLICATION

(12)

(21) Application number: **90104706.8**

(22) Date of filing: **13.03.90**

(51) Int. Cl.5: **G01N 33/68, //G01N33/574**

(30) Priority: **15.03.89 JP 64371/89**

(43) Date of publication of application:
**19.09.90 Bulletin 90/38**

(84) Designated Contracting States:
**AT BE CH DE DK ES FR GB GR IT LI LU NL SE**

(71) Applicant: **TAKEDA CHEMICAL INDUSTRIES, LTD.**
**3-6, Doshomachi 2-chome Chuo-ku**
**Osaka 541(JP)**

(72) Inventor: **Sato, Yuji**
**1-308, 4 Sumiyoshi-cho**
**Shinjuku-ku, Tokyo 162(JP)**
Inventor: **Friesen, Henry,G.**
**11 Longfellow Bay**
**Winnipeg, Manitoba(CA)**

(74) Representative: **von Kreisler, Alek,**
**Dipl.-Chem. et al**
**Patentanwälte Von Kreisler-Selting-Werner**
**Deichmannhaus am Hauptbahnhof**
**D-5000 Köln 1(DE)**

(54) **Method for detecting and measuring FGF and for diagnosis of tumor.**

(57) A sandwich method, in which an antibody coupled to a carrier, a sample and heparin labeled with a labeling agent, brings about a high sensitivity for detecting and/or measuring FGF, and the method is usable for diagnosis of tumor.

EP 0 387 777 A2

## METHOD FOR DETECTING AND MEASURING FGF AND FOR DIAGNOSIS OF TUMOR

The present invention relates to a method for detecting and/or measuring fibroblast growth factor (hereinafter also abbreviated FGF) using a sandwich technique, and to a method for diagnosis of tumor.

There are two types of FGF: basic FGF, which has a basic isoelectric point, and acidic FGF, which has an acidic isoelectric point; the entire amino acid sequence of both types is known [F. Esch et al., Proc. Natl. Acad. Sci. USA, 85, 6507(1985); K.A. Thomas et al., Proc Natl. Acad. Sci. USA, 82, 6409 (1985)].

FGF exhibits a growth promoting activity on 3T3 cells and mesoderm-derived cells, including vascular endothelial cells, in vitro and an angiogenic activity in vivo [D. Gospodarowicz et al., Endocrine Reviews, 8, 95(1987)]. In particular, the angiogenic activity of FGF, in combination with the cell growth promoting activity, can be used in therapeutic drugs for wounds and burns, and preventive/therapeutic drugs for thrombosis, arteriosclerosis and other diseases.

FGF is found in nature in only extremely small quantities; it has been very difficult, due to various limitations, to detect natural FGF from human tissues. In addition, no method has yet been developed which permits easy quantitative determination of FGF.

For these reasons, much information on FGF, indispensable for developing FGF as a pharmaceutical, remains unknown.

Obtaining information on FGF, e.g. its production and distribution mechanisms in vivo, will therefore facilitate development of FGF as a pharmaceutical.

Also, accurate quantitative determination of FGF is important in purifying this protein from transformed cells. Furthermore, it is very important to monitor blood FGF levels in FGF-administered animals; however, such measurement is impossible by conventional methods, all of which use 3T3 cells, due to sample contamination with blood serum. Conventionally, FCF measurement has been achieved by cultivating 3T3 cells at reduced serum concentrations to suppress DNA synthesis, adding FGF to the cells, and calculating the degree of recovery of DNA synthesis capability. This method, however, is faulty in that the use of cells necessitates delicate micromanipulation and produces wide errors of measurement, and that much time is taken to obtain results. It has therefore been hoped that a simple and reliable method of measuring FGF would be developed to accomplish these purposes. Furthermore, it has been desired to readily determined whether a patient has a tumor or tumors.

In the light of these circumstances, the present inventors conducted various investigations with the aim of finding a practical means of FGF measurement, and found that FGF can be measured to high sensitivity by using heparin as a ligand labeled with a labeling agent in the sandwich technique wherein two ligands are used. The present inventors made further investigations based on this finding, in developing the present invention.

Accordingly, the present invention involves (1) a method of detecting and/or measuring FGF by a sandwich technique using an antibody coupled to a carrier, a sample and heparin labeled with a labeling agent, and (2) use of an antibody coupled to a carrier and heparin labeled with a labeling agent .for diagnosing tumor by subjecting a sample to a sandwich method.

The method of detecting and measuring the present invention may be based on radioimmunoassay (hereinafter also abbreviated RIA) or enzyme immunoassay (hereinafter also abbreviated EIA).

The FGF may be any FGF, as long as it is derived from warm-blooded animals, and may also be an FGF mutein.

Therefore, in the present specification, FGF includes its mutein, unless otherwise stated.

Both types of FGF, i.e. acidic FGF (hereinafter also abbreviated aFGF) and basic FGF (hereinafter also abbreviated bFGF) are included; basic FGF however is preferred.

The FGF may be of natural derivation, or may be produced by gene engineering technology.

Examples of mammalian-derived aFGF include bovine aFGF [K.A. Thomas et al., Proc. Natl. Acad. Sci. USA, 81, 357(1984)] and human aFGF (G. Gimenez-Gallego et al., Biochem. Biophys. Res. Commun., 138, 611 (1986)].

Examples of mammalian-derived bFGF include bovine bFGF [Proc. Natl. Acad. Sci. USA, 82, 6507-(1985)] and human bFGF [European Patent Publication No. 237966; European Molecular Biology Organization (EMBO) Journal,5,2523(1986)].

The above-mentioned mutein refers to a peptide or protein with a mutated amino acid sequence; the mutation includes amino acid addition, constituent amino acid deletion and amino acid replacement by other amino acid.

Examples of amino acid addition include addition of at least one amino acid.

Examples of constituent amino acid deletion include a lack of at least one FGF-constituent amino acid.

Examples of amino acid replacement by other amino acid include replacement of at least one FGF-constituent amino acid by other amino acid.

The "at least one amino acid" in the mutein resulting from the addition of at least one amino acid to FGF does not include methionine derived from the initiation codon or signal peptide used for peptide expression.

Any number of amino acids may be added, as long as the properties of FGF are maintained. Examples of preferred amino acids to be added include partial or entire amino acid sequences of proteins recognized as homologous with FGF and having similar activities.

In the mutein resulting from a deletion of at least one constituent amino acid from FGF, any number of constituent amino acids may be lacking, as long as the properties of FGF are maintained.

In the mutein resulting from the replacement of at least one FGF-constituent amino acid by another, any number of FGF-constituent amino acids may be so replaced, as long as the properties of FGF are maintained.

Examples of constituent amino acids to be replaced include cysteine and others; cysteine however is preferred. Examples of constituent amino acids to be replaced, other than cysteine, include aspartic acid, arginine, glycine and valine.

When the constituent amino acid to be replaced is cysteine, it is preferable that the replacement amino acid be e.g. a neutral amino acid. Examples include glycine, valine, alanine, leucine, isoleucine, tyrosine, phenylalanine, histidine, tryptophane, serine, threonine and methionine; serine and threonine however are preferred.

When the constituent amino acid to be replaced is not cysteine, it is necessary to choose a replacement amino acid different from the constituent amino acid to be replaced, in such properties as hydrophilicity, hydro phobicity or electric charge. Specifically, when the amino acid to be replaced is aspartic acid, examples of the replacement amino acid include asparagine, threonine, valine, phenylalanine, and arginine; asparagine and arginine however are preferred.

When the amino acid to be replaced is arginine, examples of the replacement amino acid are glutamine, threonine, leucine, phenylalanine and aspartic acid; glutamine however is preferred.

When the constituent amino acid to be replaced is glycine, examples of the replacement amino acid include threonine, leucine, phenylalanine, serine, glutamic acid and arginine; threonine however is preferred.

When the constituent amino acid to be replaced is serine, examples of the replacement amino acid include methionine, alanine, leucine, cysteine, glutamine, arginine, and aspartic acid; methionine however is preferred.

When the constituent amino acid to be replaced is valine, examples of the replacement amino acid include serine, leucine, proline, glycine, lysine, and aspartic acid; serine however is preferred.

The preferred constituent amino acids to be replaced are aspartic acid, arginine, glycine, serine, and valine.

The preferred replacement amino acids are asparagine, glutamine, arginine, threonine, methionine, serine, and leucine.

In the replacement mutein, the constituent amino acid cysteine should ideally be replaced by serine.

In the above-mentioned replacement, two or more constituent amino acids may be replaced simultaneously; it is preferable that 2 or 3 constituent amino acids be replaced.

The mutein of the present invention may result from a combination of 2 or 3 of the above additions, eliminations and replacements.

Site-directed mutagenesis may be employed to produce said mutein. This technology is widely known and described by R.F. Lather and J.P. Lecoq in "Genetic Engineering", Academic Press (1983), pp. 31-50. Oligonucleotide-directed mutagenesis is described by M. Smith and S. Gillam in "Genetic Engineering: Principles and Methods", Prenam Press (1981), vol. 3, pp 1-32.

To produce the structural gene which encodes said mutein:

(a) single-stranded DNA comprising a single strand of the FGF structural gene is hybridized with a mutant oligonucleotide primer (this primer is complementary to the region containing, for example, a cysteine codon to be replaced by the single strand, or, as the case may be, an antisense triplet which forms a pair with this codon, except for the discrepancy from amino acid encoding codons other than the above-mentioned codon, or, as the case may be, antisense triplets),

(b) the primer is elongated using DNA polymerase to form a mutagenic heteroduplex, and

(c) this mutagenic heteroduplex is replicated.

The phage DNA carrying the mutated gene is then isolated and inserted into a plasmid.

The plasmid thus prepared is used to transform an appropriate host; the transformant thus obtained is cultivated in medium to produce the desired mutein.

Examples of the muteins include those described in Biochemical and Biophysical Research Communications, vol. 151, pp. 701-708 (1988), European Patent Publication No. 281,822.

The method of the present invention is based on the principle of the sandwich technique, a noncompetitive method of immunochemical measurement.

Examples of carriers useful for the method of the present invention include gel grains such as agarose gels [eg. Sepharose 4B, Sepharose 6B (produced by Pharmacia Fine Chemical, Sweden)], dextran gels [e.g. Sephadex G-75, Sephadex G-100, Sephadex G-200 (produced by Pharmacia Fine Chemical, Sweden)] and polyacrylamide gels [e.g. Biogel P-30, Biogel P-60, Biogel P-100 (produced by Bio-Rad Laboratories, USA)]; cellulose grains such as Avicel (produced by Asahi Chemical Industries, Japan) and ion exchange cellulose (e.g. diethylaminoethyl cellulose, carboxymethyl cellulose); physical adsorbents such as glass (e.g. glass balls, glass rods, aminoalkyl glass balls, aminoalkyl glass rods), silicon pieces, styrene resins (e.g. polystyrene balls, polystyrene grains), and immunoassay plates (e.g. plated produced by Nunc, Sweden); and ion exchange resins such as weakly acidic cation exchange resins [eg. Amberlite IRC-50 (produced by Rohm & Haas, USA), Zeo-Karb 226 (produced by Permutit AG, West Germany)] and weakly alkaline anion exchange resins [eg. Amberlite IR-4B, Dowex 3 (produced by Dow Chemical, USA)].

The antibody against FGF, used as a conjugate for the method of the present invention, may be polyclonal or monoclonal.

The polyclonal antibody against FGF can be produced by inoculating FGF as antigen to a warm-blooded animal to produce an anti-FGF antibody, which is then collected.

Any FGF can be used as the antigen, as long as it possesses biological or immunological activities similar to those of natural FGF; examples include FGF species produced by gene engineering technology and their fragments comprising a partial amino acid sequence essential to their biological or immunological activities. These FGF species produced by gene engineering technology may have additional Met at the polypeptide amino terminal, and may also be a mixture of an FGF and another FGF having additional Met at the amino terminal.

The above fragments can be produced by known routine methods of peptide synthesis, which may be based on a solid phase method or liquid phase method. Examples of such methods of peptide synthesis include the methods described in "The Peptides", vol. 1 (1966), edited by Schröder and Lubke, Academic Press, New York, USA, "Peptide Synthesis (in Japanese)", edited by Izumiya et al., Maruzen (1975), and "Fundamentals and Experiments of Peptide Synthesis (in Japanese)", edited by Izumiya et al., Maruzen (1985).

These fragments may be produced by cleaving FGF using appropriate enzymes. Examples of such methods include the method described in "Biochemical Experiments Course 1, Protein Chemistry II (in Japanese)", edited by the Japanese Biochemical Society, Tokyo Kagaku Dojin (1976), pp. 255-332.

FGF as the antigen is coupled with carrier protein. Examples of the carrier protein include bovine tyroglobulin, bovine serum albumin, bovine gamma globulin, hemocyamin, and Freud's complete adjuvant (produced by Difco Laboratories).

Antigen FGF and carrier protein can be coupled by a routine method. Examples of coupling reagents include glutaraldehyde and water-soluble carbodiimide. The antigen FGF and the carrier protein should be coupled in a ratio of about 1 to 1 to about 1 to 10. Good results are often obtained when reaction is carried out at a nearly neutral pH level, ideally pH 6 to 8. Reaction time is generally 1 to 12 hr, preferably 2 to 6 hr. The complex thus formed may be dialyzed against water at 0 to 18°C by a routine method, after which it may be frozen or lyophilized for storage.

To produce a polyclonal antibody, the immunogen thus produced is inoculated to a warm-blooded animal. Examples of warm-blooded animals which can be used to produce the above antibody include mammals (e.g. rabbits, sheep, goats, rats, mice, guinea pigs, bovines, horses, swine) and birds (e.g. chickens, doves, ducks, geese, quails). The immunogen is inoculated to a warm-blooded animal in an amount such that efficient antibody production will occur; for example, antibodies are often produced when 1 mg of the immunogen, in an emulsion in 1 mℓ of a physiological saline solution containing Freund's complete adjuvant, is subcutaneously inoculated to a rabbit at the back and hind leg paw 5 times at 4-week intervals. The antibody thus formed in the warm-blooded animal is, for example, collected as follows: when a rabbit is used, blood is usually collected via ear vein between 7 and 12 days after the final inoculation, then centrifuged to yield a serum. The antiserum thus obtained is subjected to affinity chromatography using a carrier coupled with an antigen peptide; the fraction adsorbed to the column is recovered to separate a purified polyclonal antibody.

It is also possible to use the monoclonal antibody obtained in accordance with the method described by Milstein et al. [Nature, *256*, 495(1975)]. This monoclonal antibody is produced by immunizing a mammal with the antigen polypeptide or protein complex, fusing spleen cells excised from the animal and homo- or heterogenic lymphoid cells to yield a hybridoma, which is then cloned, inoculating the cloned hybridoma to a mammal to produce and accumulate a monoclonal antibody, which is then collected.

Immunization of e.g. mice can be achieved via any route, such as subcutaneous, intraperitoneal, intravenous, intramuscular, and intracutaneous administration; mainly subcutaneous, intraperitoneal, or intravenous (preferably subcutaneous) injection is recommended. Immunization interval immunization quantity etc. can also be widely varied so that various modes are possible; for example, in a method often used, immunization is conducted 2 to 6 times at 2-week intervals and spleen cells are excised and used 1 to 5 days, preferably 2 to 4 days after final immunization. It is desirable that the immunization quantity be over 0.1 μg, preferably 10 to 300 μg per mouse in each immunization, in terms of peptide. It is also desirable that partial blood sampling be conducted before spleen excision, to confirm increased blood antibody level, and that a cell fusion experiment be conducted using spleen cells.

For the cell fusion of spleen cells and lymphoid cells, excised mouse spleen cells are fused with a lymphoid cell line, such as an appropriate allo- or heterogenic (preferably allogenic) myeloma with a marker for hypoxanthine-guanine-phosphoribosyl transferase deficiency (HGPRT⁻) or thymidine kinase deficiency (TK⁻) [e.g. P3-X63-Ag 8UI (Ichimori et al., Journal of Immunological Method, *80*, 55 (1985)]. Fusion can, for example, be achieved in accordance with the method of Köller and Milstein [Nature, *256*, 495 (1975)]. For example, myeloma cells and spleen cells, in a ratio of about 1 to 5, are suspended in a medium prepared by mixing Iskov medium and Ham F-12 medium in a 1 to 1 ratio (hereinafter referred to as IH medium), followed by cell fusion reaction in the presence of a fusogen such as Sendai virus or polyethylene glycol (PEG). Dimethylsulfoxide (DMSO) and other fusion promoters can also be added. PEG with a degree of polymerization of 1000 to 6000 is normally used at a concentration of 10 to 80% for 0.5 to 30 minutes: efficient fusion is achieved, for example, by treating PEG 6000 at 35 to 55% for 4 to 10 minutes. The fused cells can be selectively proliferated using hypoxanthine-aminopterin-thymidine medium (HAT medium; Nature, *256*, 495 (1975)) etc.

The culture supernatant of the proliferated cells can be screened for the desired antibody production; antibody titer screening can be conducted as follows: The culture supernatant is first examined for the production of antibody against the immunogen peptide by radioimmunoassay (RIA) or enzyme immunoassay (EIA). Various modifications are possible for these methods. An example of a preferred method of measurement based on EIA is described below. A carrier, such as cellulose beads, is coupled with e.g. rabbit anti-mouse immunoglobulin antibody by a routine method; the subject culture supernatant or mouse serum is added, followed by reaction at constant temperature (about 4 to 40°C; the same applies below) for a given time. The reaction product is then thoroughly washed; an enzyme-labeled peptide (enzyme and peptide are coupled by a routine method, then purified) is added, followed by reaction at constant temperature for a given time. After the reaction product is thoroughly washed, an enzyme substrate is added, followed by reaction at a constant temperature for a given time; the formed coloring substance is then measured by optical absorbance, fluorescence intensity etc.

It is desirable that cells which proliferate in the selection medium in the wells, and in which antibody activities against the immunogen peptide are noted, be cloned by limited dilution analysis etc. The supernatant of the cloned cells is screened in the same manner to increase the number of cells with high antibody titer, to yield a hybridoma clone which produces a monoclonal antibody against the immunogen peptide.

The hybridoma thus cloned is proliferated in liquid medium. Specifically, the desired monoclonal antibody can be obtained from the culture broth obtained by cultivating the hybridoma in a liquid medium, such as a medium prepared by adding about 0.1~40% bovine serum to RPMI-1640 medium [Moore, G.E. et al., Journal of American Medical Association, *199*, 549 (1967)] for 2 to 10 days, preferably 3 to 5 days. The antibody can also be obtained by intraperitoneally inoculating the hybridoma to a mammal for cell proliferation, and collecting the ascites fluid. For this purpose, when mice are used, for example $1 \times 10^4$ to $1 \times 10^7$, preferably $5 \times 10^5$ to $2 \times 10^6$ hybridoma cells are intraperitoneally inoculated to each mouse of BALB/c or other lines, previously inoculated with mineral oil etc.; ascites fluid is then collected 7 to 20 days, preferably 10 to 14 days after inoculation. The antibody produced and accumulated in the ascites fluid can easily be isolated in the form of pure immunoglobulin by e.g. ammonium sulfate fractionation and DEAE-cellulose column chromatography.

The antibody molecule may be IgG, or its fragment [e.g. F(ab′)₂, Fab′, Fab″, or Fab]. It is preferable however that the antibody molecule is coupled directly with a labeling agent be Fab′.

To couple an antibody to the carrier, known routine methods can be used. Examples of usable methods include the bromocyan method and glutaraldehyde method, both described in "Taisha," vol. 8(1971), p. 696. In a simple procedure, physical adsorption onto the carrier's surface may be employed. For example, the antibody is immobilized onto a carrier such as a 96-well plastic plate (e.g. Immunoplate, produced by Nunc, Denmark), at 0.1 to 10 $\mu$g/well, glass beads and plastic beads. Immobilization is achieved by an overnight reaction (at about 4°C), or at room temperature for 0.5 to 4 hours, in the case of plastic carriers. In the case of glass carriers, immobilization is achieved, for example, by the method described in Proc. Natl. Acad. Sci. USA, vol. 80, pp. 3513-3516 (1983). Various commercially available plates for antibody immobilization (e.g. the Immunoplate, produced by Nunc, mentioned above) can also be used.

Examples of heparins used for the method of the present invention include N-sulfuric acid-, N-acetyl-, and O-sulfuric acid-substituted products of polysaccharides comprising D-glucosamine, D-glucuronic acid and L-iduronic acid.

Examples of labeling agents for RIA include $^3$H, $^{125}$I and other radioisotopes.

Examples of labeling agents for EIA include enzymes, fluorescent substances and luminescent substances; the use of enzyme is recommended. Stable enzymes with high specific activity are preferred; peroxidase, alkaline phosphatase, $\beta$-D-galactosidase, glucose oxidase etc. can be used; peroxidase is preferred. Peroxidase of various derivations can be used; examples include peroxidase derived from horseradish, pinapple, fig, sweet potato, kidney bean, corn etc.; horseradish peroxidase (HRP) is preferred.

Examples of fluorescent substances include FITC (fluorescein isothiocyanate), rhodamine, and lanthanide chelate.

Examples of luminescent substances include acridinium esters. For coupling heparin with a labeling agent, a heparin molecule which is highly reactive on peptide or protein is first chosen. This coupling can, for example, be achieved with the method described by A.D. Cardin et al. [Thrombosis Research, *34*, 541 (1984)], which uses low density lipoprotein (LDL)-AffiGel-10. The highly reactive heparin (HRH) (purified by this method) is then coupled with a labeling agent. Examples of labeling agents include $^{125}$I. Before coupling $^{125}$I to HRH, it is necessary to maximize the specific activity for labeling. This can, for example, be achieved by introducing a hydroxyphenyl group to HRH. This particular maximization can, for example, be achieved by the method using the Bolton-Hunter reagent, described by N. Hirose et al. [Analytical Biochemistry, *156*, 320 (1986)].

To introduce $^{125}$I to the hydroxyphenyl-HRH thus obtained, methods known in the art can be used, including the method using an iodogen, described by R.H. Raja et al. [Anal. Biochem., *139*, 168(1984)].

The subject sample to be measured, using the measurement system of the present invention, includes: urine, serum, plasma, cerebrospinal fluid and other body fluids, or cell or bacterial extracts or their supernatants.

An example of EIA for the measuring method of the present invention, when using peroxidase as the labeling agent, is described in detail below; however, such use of peroxidase is not to be construed as a limitation on the present invention.

① The FGF-containing subject sample is transferred to the carrier-coupled antibody in order to couple the FGF to the antibody, and is then reacted with the peroxidase-coupled heparin.

② A peroxidase substrate is added to the reaction product obtained in ①; the photo-absorbance or fluorescence intensity of the resulting substance is then determined, in order to calculate the enzyme activity of the above reaction product.

③ Separately, procedures ① and ② above are repeated with a known amount of FGF standard solution in order to draw a standard curve showing the relationship between FGF and the absorbance or fluorescence intensity.

④ The absorbance or fluorescence intensity obtained for the subject of analysis (subject sample), which contains an unknown amount of FGF, is applied to the standard curve to determine the amount of FGF in the subject of analysis.

An example of RIA for the measuring method of the present invention, when using $^{125}$I as the labeling agent, is described in detail below; however, such use of $^{125}$I is not to be construed as a limitation on the present invention.

① The FGF-containing subject sample is transferred to the carrier-coupled antibody in order to couple the FGF to the antibody, and is then reacted with heparin (hydroxyphenyl-HRH).

② The $\gamma$-radioactivity of the reaction product obtained in ① is determined.

③ Separately, procedures ① and ② above are repeated with a known amount of FGF standard solution in order to draw a standard curve showing the relationship between FGF and $\gamma$-radioactivity.

④ The $\gamma$-radioactivity obtained for the subject of analysis (subject sample), which contains an unknown amount of FGF, is applied to the standard curve to determine the amount of FGF in the subject of analysis. This method is expected to provide an efficient means of detecting and measuring FGF in clinical specimens (e.g. blood, serum, plasma, urine, ascites, cystic fluid, pleural effusion, cerebrospinal fluid), and an efficient means of investigating the distribution of FGF in tissues and organs.

Using heparin in the form of a conjugate coupled with a labeling agent, the method of the present invention offers high measurement sensitivity, thus permitting detection and measurement of trace amounts of blood FGF.

The method of detecting and measuring FGF using the present invention is also applicable to diagnosis of tumors. Since tumor cells are believed to release large amounts of FGF, the blood, urine, ascites, pleural effusion, cerebrospinal fluid, external effusion, gastric juice, cystic fluid and other body fluids of tumor patients should contain more FGF than do those of normal humans. Therefore, if a large amount of FGF is detected in the subject sample obtained from a patient, the patient's disease can be primarily diagnosed as a tumor.

As stated above, the method of tumor diagnosis according to the present invention, is characterized by the detection and measurement of a fibroblast growth factor (FGF) by the sandwich technique (using a carrier-coupled antibody, a subject sample and heparin coupled with a labeling agent). This diagnosis method permits the primary diagnostic screening of patients for tumors.

Examples of target tumors include: brain tumor, lung tumor, digestive tract tumor, liver tumor, pancreas tumor, gallbladder tumor, bile duct tumor, kidney tumor, urinary bladder tumor, adrenal tumor, rhabdomyoma, rhabdomyosarcoma, leiomyosarcoma, endocrine neoplasia tumor, parathyroid tumor, and parathyroid cancer.

Detection and measurement can be achieved in the same manner as the above-mentioned methods.

Accordingly, by comparing the amount of FGF in a preselected sample and comparing that sample with a standard for a "normal" sample, the presence of a tumor can be determined. This, as indicated above, would occur when a greater amount of FGF is present in the preselected sample than in the "normal" control. Preferably, the sample should contain at least double the amount of FGF, more preferably, it should contain at least three times the amount of FGF than the "normal" control contains.

The FGF-detecting and/or measuring method of the present invention permits measurement of FGF to high sensitivity, thus the present method functions well as a means of detecting and measuring FGF in body fluids or cells.

Abbreviations for amino acids etc. used in the present specification are based on abbreviations specified by the IUPAC-IUB Commission on Biochemical Nomenclature or those commonly used in relevant fields; some examples are given below. Note that when an optical isomer of amino acid is present, it is an L-body, unless otherwise stated.

Tyr: Tyrosine
Nle: Norleucine

The present invention will now be described in more detail by means of the following examples, although these are not to be construed as limiting the present invention.

Brief Explanation of the Drawings

Fig. 1 shows the results of western blotting of the anti-[Tyr$^{11}$] human bFGF (1-11) Nle$^{12}$ rabbit antibody, obtained in Reference Example 1.

Fig. 2 shows the standard curve of RIA using recombinant-derived bovine bFGF, obtained in Example 1 (2).

Fig. 3 shows the effect of serum on measurement of bFGF, determined in Example 1 (2).

Fig. 4 shows the amount of bFGF in patient's blood, determined in Example 2.

Reference Example 1

(1) Preparation of anti-[Tyr$^{11}$] human bFGF (1-11) Nle$^{12}$ rabbit antibody

The peptide [Tyr$^{11}$] human bFGF (1-11) Nle$^{12}$ [produced in the same manner as described in "Peptide Synthesis", M. Bodanszky, Y.S. Klausner, and M. A. Ondetti, John Weily and Sons, N. Y. (1976); purchased from Alberta Peptide Institute, Edmonton Alberta, Canada] was bound with KLH (Keyhole Limpet Hemocyanin) using glutaraldehyde; this conjugate was dissolved in a phosphate buffer solution (PBS) (8000 mg/$\ell$ NaCl, 200 mg/$\ell$ KCl, 2160 mg/$\ell$ NaHPO$_4 \cdot$7H$_2$O, 200 mg/$\ell$ KH$_2$PO$_4$, 100 mg/$\ell$ MgCl$_2 \cdot$6H$_2$O) to a peptide concentration of 100 $\mu$g/m$\ell$; this solution was mixed with an equal amount of Freund's complete adjuvant (Difco Laboratories). 1 m$\ell$ of this mixture was intradarmally administered to rabbits; a mixture of 100 $\mu$g/m$\ell$ of the above-mentioned synthetic peptide and an equal amount of incomplete adjuvant (Difco

7

Laboratories) was then administered at 2-week intervals. After 5 administrations, blood was collected and kept standing at room temperature for 5 hr, then left at 4°C for 16 hr, after which it was centrifuged at 3000 rpm for 10 min; the supernatant was used as anti-[Tyr¹¹] human bFGF (1-11) Nle¹² rabbit serum. Also, 0.5 g of cyanogen bromide-activated Sepharose 4B (Pharmacia) was transferred to a glass filter and sufficiently expanded with 20 mℓ of 0.001 N HCl, after which it was thoroughly washed with 400 mℓ of the same aqueous hydrochloric acid. Separately, 2 mℓ of a solution of 0.1 M sodium bicarbonate and 0.5 M sodium chloride was added to 100 μℓ of a solution of the peptide [Tyr¹¹] human bFGF (1-11) Nle¹² (protein content, 40 mg/mℓ); this mixed solution was adjusted to pH 8.4 with a solution of 0.1 M sodium hydroxide. To this solution, the above-mentioned cyanogen bromide-activated Sepharose 4B was added, followed by reaction at room temperature for 2 hr. After reaction, the gel was transferred to a glass filter and washed with 100 mℓ of a solution of 0.1 M sodium bicarbonate and 0.5 M sodium chloride. The gel was then added to 10 mℓ of a solution of 0.1 M Tris-HCl, pH 8.0; reaction was carried out at 4°C for 20 hr while stirring the solution gently to mask the remained active groups. The gel was then washed with sequential additions of a 0.1 M acetate buffer solution containing 1 M sodium chloride (pH 4.0) and a 0.1 M borate buffer solution containing 1 M sodium chloride (pH 8.0), each in 100 mℓ amounts. The gel was then suspended in PBS and stored in a column at 4°C.

The above-mentioned serum was applied to this column; the column was then thoroughly washed with PBS so that optical absorption at 280 nm disappeared. Elution was then conducted with a 0.2 M glycine-HCl buffer solution (pH 2.3) containing 10% dioxane; the eluted fraction was immediately neutralized with a one-third its amount of 1 M Tris-HCl, pH 7.4. This solution was dialyzed against PBS at 4°C for 16 hr, after which it was centrifuged at 10,000 rpm for 10 min; the supernatant was used as anti-[Tyr¹¹] human bFGF (1-11) Nle¹² rabbit antibody.

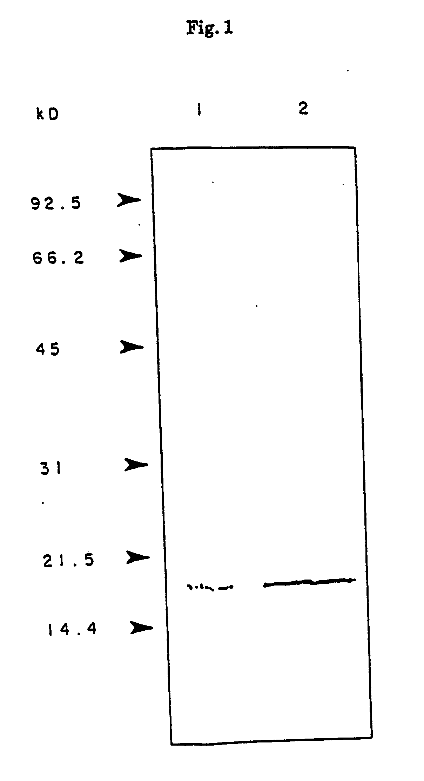

To evaluate the specificity of the antibody, the heparin-coupled protein in the culture supernatant of bovine corneal endothelial cells (BCE cell) and recombinant bFGF was subjected to SDS-PAGE, followed by western blotting; a specific band (lane 1) was detected at the position corresponding to bFGF (lane 2) (see Fig. 1).

<u>Example 1</u>

(1) Preparation of ¹²⁵I-labeled heparin:

Heparin was labeled with ¹²⁵I in accordance with the method described in Analytical Biochemistry, *156*, 320-325 (1986), which is as follows:

Heparin was purified using a column of low density lipoprotein (LDL)- AffiGel-10 in order to obtain HRH (highly reactive heparin), which was then reacted with Bolton-Hunter reagent to introduce a hydroxyphenyl group. Iodization with ¹²⁵I (1 mCi), was conducted in a test tube containing 15 μg of solidified iodogen, cooled with ice for 15 minutes. Unreacted ¹²⁵I was removed by dialysis against distilled water, to obtain purified ¹²⁵I-heparin.

(2) Two-site RIA using ¹²⁵I-heparin

The following procedures were followed using a 96-well microtiter plate (Dynatech Laboratories). The plate was washed with a physiological saline solution/0.1% Tween 20, rinsed with distilled water, treated with ethanol, and then thoroughly washed with distilled water to eliminate well-to-well differences.

The anti-[Thr¹¹] human bFGF (1-11) Nle¹² rabbit antibody (3 μg/mℓ) (obtained in Reference Example 1) was added to the plate at 100 or 200 μℓ per well. The plate was left at 4°C overnight. After discarding the solution, the plate was washed with PBS; 300 μℓ of 1% BSA (bovine serum albumin)/PBS/0.04% NaN₃ was added to each well; the plate was left at 4°C, for at least one night, to facilitate blocking.

After discarding the solution in the plate, the sample, appropriately diluted and adjusted to an NaCl concentration of 0.5 M, was added to the plate and left at 4°C overnight.

Alter sample adsorption to the plate, each well was washed with 0.5 M NaCl/PBS; the ¹²⁵I-heparin described in (1) above was added to the plate at 10 to 20 × 10⁴ cpm per well; the plate was left at 4°C overnight. After thorough washing with 0.5 M NaCl/PBS, each well was cut out using scissors; the count from each well was taken using a gamma counter.

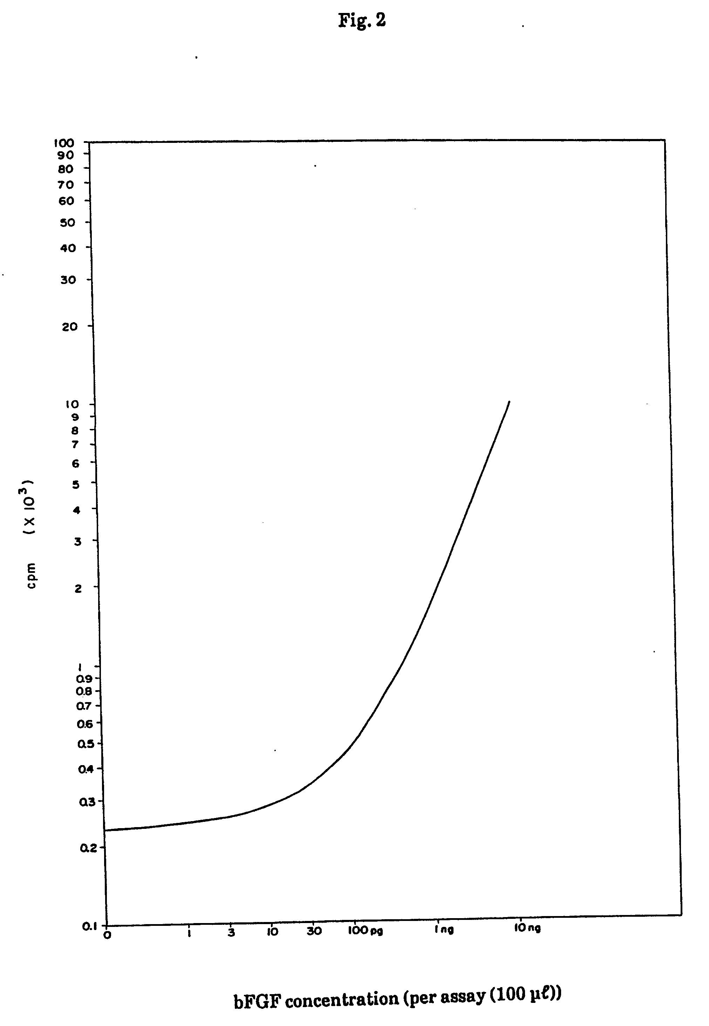

The standard curve obtained by this RIA procedure, using recombinant-derived bovine bFGF, is shown in Fig. 2 (n = 3, m±SE). This curve shows that about 1 to 10 pg (lower detection limit) of bFGF can be detected by the present RIA procedure. Note that the recombinant-derived bovine bFGF was purchased from Amgen Co., USA.

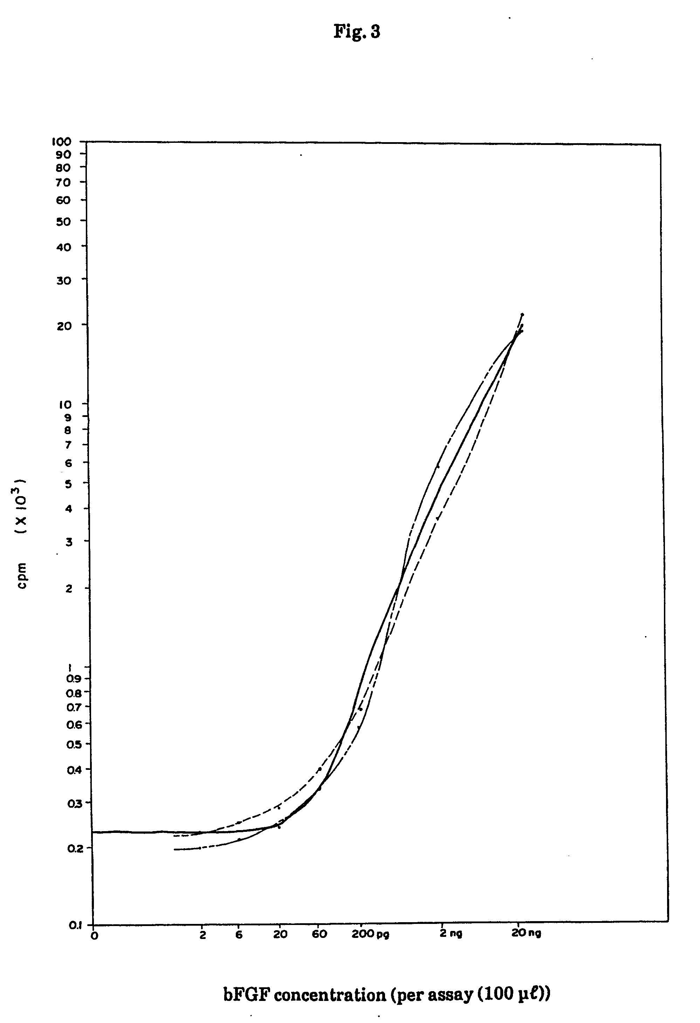

Also, the effect of serum of this RIA procedure was rated in the same manner as above. The results are shown in Fig. 3. In Fig. 3, — · · —,

, and ------- respectively show the results for 0.1% FCS, 30% FCF, and 50% FCS.

The results shown in Fig. 3 have revealed that 5%, 10%, 20%, 30%, or 50% fetal calf serum (FCS) hardly affects the quantitative determination of blood bFGF.

Also, the same RIA procedure as above was used to determine the blood bFGF concentration in patients with multiple endocrine neoplasia type 1 and normal humans. The results are shown in Table 1.

Table 1

|  | Normal humans | Patients |
|---|---|---|
| In the presence of EDTA | 0 | 92 pg/ml |
| 65% ammonium sulfate precipitation | 0 | 13 pg/ml |
| Elution from heparin column (0.2 M NaCl) | 0 | 0 |
| Elution from heparin column (0.3 M NaCl) | 0 | 0 |

Example 2

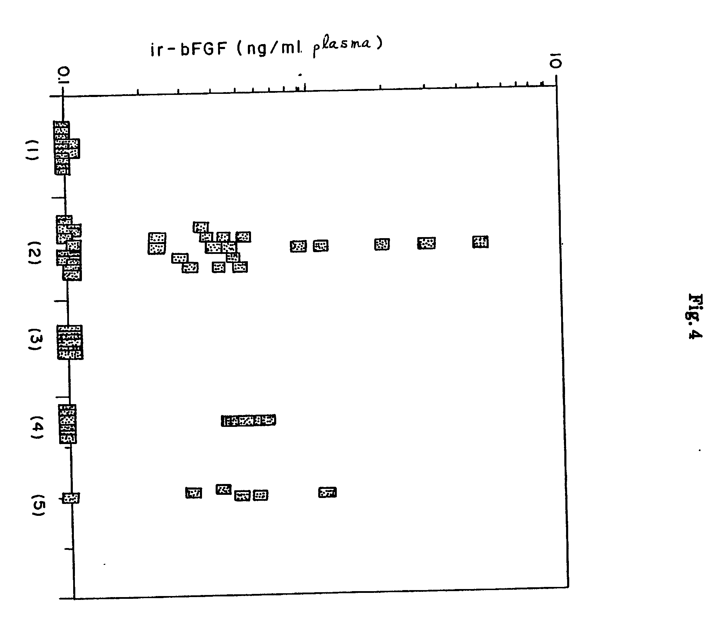

Using the same method of bFGF determination as in Example 1, blood bFGF was quantified in patients with endocrine neoplasia, sporadic parathyroid adenoma, and parathyroid carcinoma. The results are shown in Fig. 4. In Fig. 4, (1) shows the results for normal humans (n = 11), (2) for patients with familial multiple endocrine neoplasia type 1 (n = 37), (3) for relatives of patients with familial multiple endocrinoma, presenting no symptoms (n = 12), (4) for patient with sporadic parathyroid adenoma (n = 12), and (5) for patients with parathyroid carcinoma (n = 6). Fig. 4 reveals that the blood bFGF concentration is evidently high in these patients. These results suggest an applicability of the present method of bFGF quantitative determination for tumor diagnosis.

## Claims

1. A method for detecting and/or measuring fibroblast growth factor (FGF), which comprises subjecting a sample to a sandwich technique using an antibody coupled to a carrier and heparin labeled with a labeling agent.

2. A method as claimed in Claim 1, wherein the FGF is a basic FGF.

3. A method as claimed in Claim 2, wherein the basic FGF is human basic FGF.

4. Use of an antibody coupled to a carrier and heparin labeled with a labeling agent for diagnosing tumor by subjecting a sample to a sandwich method.

5. Use as claimed in Claim 4, wherein the FGF is a basic FGF

6. Use as claimed in Claim 5, wherein the basic FGF is human basic FGF..

EP 0 387 777 A2

Fig. 1

Fig. 2

bFGF concentration (per assay (100 μℓ))

## Fig. 3

bFGF concentration (per assay (100 μℓ))

**Fig. 4**